# EUROPEAN PATENT SPECIFICATION

(11) **EP 1 695 985 B1**
(45) Date of publication and mention of the grant of the patent: **09.03.2011**
(21) Application number: 05077948.7
(22) Date of filing: 03.04.1998
(51) Int. Cl.: C07K 16/00, C12N 15/13, C12N 15/73, A61K 39/395

(54) **Methods for forming humanised antibodies by random mutagenesis**
Verfahren zur Herstellung humanisierter Antikörper durch randomisierte Mutagenese
Procédé pour la production d'anticorps humanisés par mutagénèse randomisé

(30) Priority: 07.04.1997 US 833504
(43) Date of publication of application: 30.08.2006
(62) Divisional of application: 98918013.8
(73) Proprietor: Genentech, Inc., South San Francisco CA 94080-4990 (US)
(72) Inventor: Wells, James A., Burlingame, CA 94010 (US); Baca, Manuel, Foster City, CA 94404 (US); Presta, Leonard G., San Francisco, CA 94109 (US)
(74) Representative: Wichmann, Hendrik

(56) References cited:
- WO-A-92/18619
- WO-A-92/22653
- WO-A-94/04679
- GB-A- 2 268 744
- FOOTE J ET AL: "ANTIBODY FRAMEWORK RESIDUES AFFECTING THE CONFORMATION OF THE HYPERCARIABLE LOOPS" JOURNAL OF MOLECULAR BIOLOGY, LONDON, GB, vol. 224, no. 2, January 1992 (1992-01), pages 487-499, XP000564649 ISSN: 0022-2836
- HAWKINS R E ET AL: "SELECTION OF PHAGE ANTIBODIES BY BINDING AFFINITY MIMICKING AFFINITY MATURATION" JOURNAL OF MOLECULAR BIOLOGY, LONDON, GB, vol. 226, no. 3, 1992, pages 889-896, XP000940994 ISSN: 0022-2836
- CLACKSON T ET AL: "MAKING ANTIBODY FRAGMENT USING PHAGE DISPLAY LIBRARIES" NATURE, NATURE PUBLISHING GROUP, LONDON, GB, vol. 352, 15 August 1991 (1991-08-15), pages 624-628, XP001019232 ISSN: 0028-0836
- KUNKEL T A ET AL: "Efficient site-directed mutagenesis using uracil-containing DNA." METHODS IN ENZYMOLOGY. 1991, vol. 204, 1991, pages 125-139, XP009073170 ISSN: 0076-6879
- M. BACA ET AL.,: "Antibody humanization using monovalent phage display" JOURNAL OF BIOLOGICAL CHEMISTRY, vol. 272, no. 16, 18 April 1997 (1997-04-18), pages 10678-10684, XP002077471

## Description

### FIELD OF THE INVENTION

The present invention is directed at methods for preparing humanized antibodies. In particular, the present invention is directed at methods for preparing humanized antibodies using a monovalent phage display system and antibody mutants produced by random mutagenesis of a small set of critical framework residues made to a single human framework. More particularly, this invention is directed at the humanization of a murine antibody which binds to vascular endothelial growth factor (VEGF).

### BACKGROUND OF THE INVENTION

Monoclonal antibodies (mAbs) have enormous potential as therapeutic agents, particularly when they can be used to regulate defined systems. For example, in some circumstances it would be desirable to regulate a system such as angiogenesis, where new blood capillaries are formed from the walls of existing small vessels. Angiogenesis is generally important after infliction of a wound or infection so that a burst of capillary growth can be stimulated in the neighborhood of the damaged tissue. However, angiogenesis is also important in tumor growth since, for continued growth, a tumor must induce the formation of a capillary network that invades the tumor mass.

Certain growth factors have been identified which regulate angiogenesis. Of particular interest is the vascular endothelial growth factor (VEGF), which seems to be the agent by which some tumors acquire their rich blood supply. Molecular Biology of the Cell, 3rd Ed., Alberts et al., Garland Publishing, page 1154 (1994). Therefore, mAbs to VEGF, for example, can be useful for a variety of reasons, including for use in the regulation of angiogenesis and more particularly, as an anti-tumor agent. A murine anti-VEGF mAb A4.6.1 which blocks VEGF receptor binding has been previously described. This antibody has been shown to inhibit mitogenic signaling. Kim et al., Growth Factors 7, 53 (1992); Kim et al., Nature 362, 841 (1993).

Most mAbs including the anti-VEGF described above are derived from murine or other non-human sources which limits clinical efficacy. In particular, the body often reacts with an immunogenic response to non-human antibodies whereby the antibody is rapidly cleared from the system before any therapeutic effect can occur. In addition to the immunogenicity of non-human mAbs invoked when administered to humans, further limitations arise from weak recruitment of effector function.

As a means of circumventing these deficiencies, the antigen binding properties of non-human mAbs can be conferred to human antibodies through a process known as antibody "humanization". A humanized antibody contains the amino acid sequence from the six complementarily-determining regions (CDRs) (the antigen-binding site of the antibody molecule) of the parent or corresponding non-human mAb, grafted onto a human antibody framework. Therefore, humanization of non-human antibodies is commonly referred to as CDR grafting. The low content of non-human sequence in such humanized antibodies (~5%) has proven effective in reducing the immunogenicity and prolonging the serum half-life of the antibodies administered to humans. Inter alia, humanized monoclonal antibodies ("chimeric immunoglobulins") are disclosed in U.S. Patent No. 4,816,567.

Unfortunately, simple grafting of CDR sequences often yields humanized antibodies which bind antigen much more weakly than the parent non-human mAb. In order to restore high affinity, the antibody must be further engineered to fine-tune the structure of the antigen binding loops. This is achieved by replacing key residues in the framework regions of the antibody variable domains with the matching sequence from the parent murine antibody. These framework residues are usually involved in supporting the conformation of the CDR loops, although some framework residues may themselves directly contact the antigen. Studies have been conducted which note the importance of certain framework residues to CDR conformation and a comprehensive list of all the framework residues which can affect antigen binding has been compiled. Chothia et al., J. Mol. Biol. 224, 487 (1992); Foote et al., J. Mol. Biol. 224, 489 (1992). The comprehensive list includes some thiry "vernier" residues which can potentially contribute to CDR structure. Although higher antigen affinity would likely result from editing the entire set of vernier residues within a humanized antibody so as to match the corresponding parent non-human sequence, this is not generally desirable given the increased risk of immunogenicity imposed by adding further elements of non-human sequence. Thus, from a therapeutic standpoint, it is preferable to confine framework changes to the minimum set which affords a high affinity humanized antibody.

Therefore, it is desirable to identify a small set of changes which suffice to optimize binding, however, the required changes are expected to differ from one humanized antibody to the next. To achieve the desired result, one approach has been to identify the proper combination of mutations by constructing a panel of mutants having "suspect" framework residues replaced by their murine counterpart. These variants are each individually formed and tested for antigen and then combined with other variants found to have favorable binding affinities. However, this method involves cycles of individual site-directed mutagenesis, isolation and screening, and is therefore undesirable because it is time consuming and tedious.

As a means of simplifying antibody humanization, a number of different approaches have been developed. See, for example, Queen et al., PNAS USA 86, 10029 (1989); Kettleborough et al., Protein Eng. 4, 773 (1991); Tempest et al., Biotechnology 9, 266 (1991); Padlan,Mol. Immunol. 28, 489 (1991); Roguska et al., PNAS USA 91, 969 (1994); Studnicka et al., Protein Eng. 7, 805 (1994); Allen et al., J. Immumol. 135, 368 (1985); Carter et al., PNAS USA 89, 4285 (1992); Presta et al., J. Immunol. 151, 2623 (1993); Eigenbrot et al., Proteins 18, 49 (1994); Shalaby et al., J. Exp. Med. 175, 217 (1992); Kabat et al., Sequences of Proteins of Immunological Interest, (5th), Public Health Service, NIH, Bethesda, MD (1991); and Rosok et al., J. Biol. Chem. 271, 22611 (1996).
It is an object of the present invention to provide a general means of rapidly selecting framework mutations which improve the binding of humanized antibodies to their cognate antigens wherein the current methods of framework optimization based on cycles of individual site-directed mutagenesis and screening are eliminated.

It is also an object to provide rapid methods of humanizing antibodies which provide antibodies with low immunogenecity and which utilize a single human framework as a generic scaffold.

### SUMMARY OF THE INVENTION

The initial humanized anti-VEGF has a framework derived from consensus sequences of the most abundant human subclasses, namely V_{L}κ subgroup I (VₗκI) and V_{H} subgroup III (V_{H}III) wherein the CDRs from non-human anti-VEGF are grafted thereon. Random mutagenesis of critical framework residues on the initial construct produced the humanized anti-VEGF described herein which has 125 fold enhanced affinity for antigen relative to the initial humanized antibody with no framework changes. A single additional mutation gave a further six fold improvement in binding. This humanized anti-VEGF can be reproduced by the method described herein or by traditional recombinant techniques given the sequence information provided herein.

Also provided herein is a method as defined in claim 1 for rapidly producing and identifying framework mutations which improve the binding of humanized antibodies to their cognate antigens. In a preferred embodiment, non-human CDRs are grafted onto a human VₗκI- V_{H}III framework. Random mutagenesis of a small set of critical framework residues is also performed followed by monovalent display of the resultant library of antibody molecules on the surface of filamentous phage. The optimal framework sequences are then identified by affinity-based selection. Optionally, the selected antibodies can be further mutated so as to replace vernier residues which sit at the V_{L}-V_{H} interface with residues which match the non-human parent antibody.

The methods described herein can be applied to any non-human antibody.

### BRIEF DESCRIPTION OF THE DRAWINGS

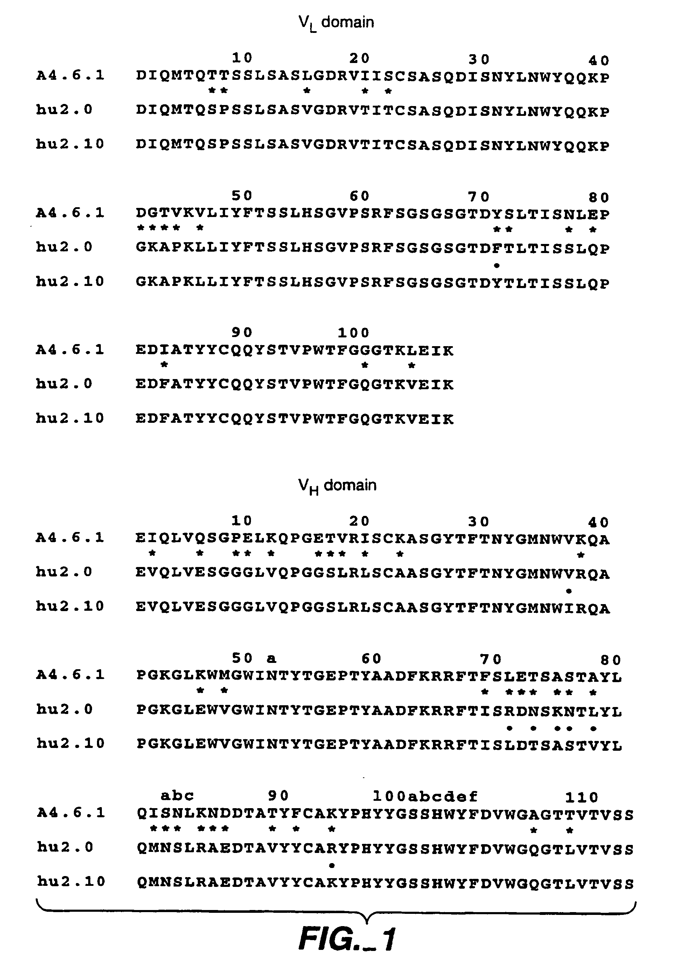

Figure 1 depicts the amino acid sequences of murine A4.6.1 (SEQ ID NO: 6 and 9 for the V_{L} and V_{H} domains, respectively), humanized A4.6.1 variant hu2.0, (SEQ ID NO: 7 and 10 for the V_{L} and V_{H} domains, respectively), and humanized A4.6.1 variant hu2.10 (SEQ ID NO: 8 and 11 for the V_{L} and V_{H} domains, respectively). Sequence numbering is according to Kabat et al., Sequences of Proteins of Immunological Interest, (5th), Public Health Service, NIH, Bethesda, MD (1991) and mismatches are indicated by asterisks (murine A4.6.1 vs hu2.0) or bullets (hu2.0 vs hu2.10). Variant hu2.0 contains only the CDR sequences (bold) from the murine antibody grafted onto a human light chain K subgroup I, heavy chain subgroup III framework. Variant hu2.10 is the consensus humanized clone obtained from phage sorting experiments described herein.

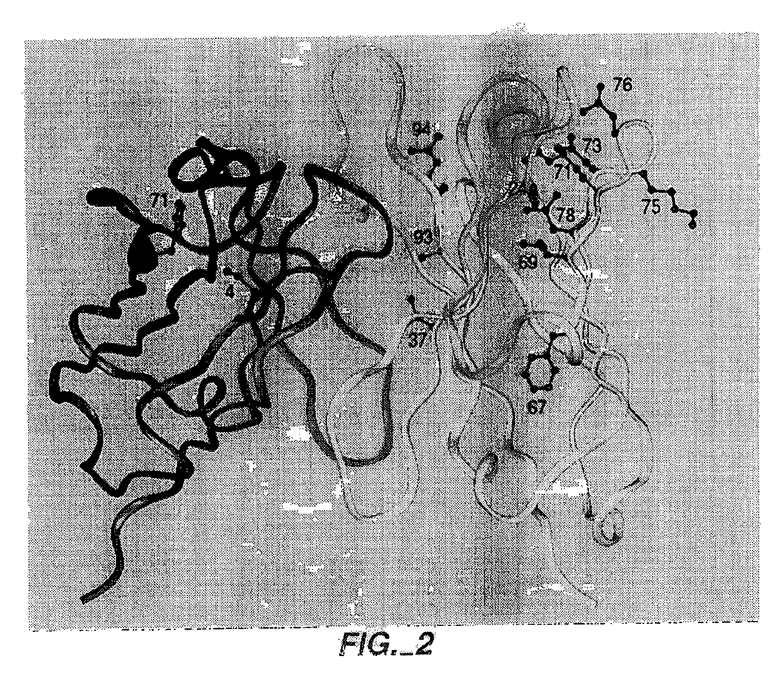

Figure 2 depicts the framework residues targeted for randomization.

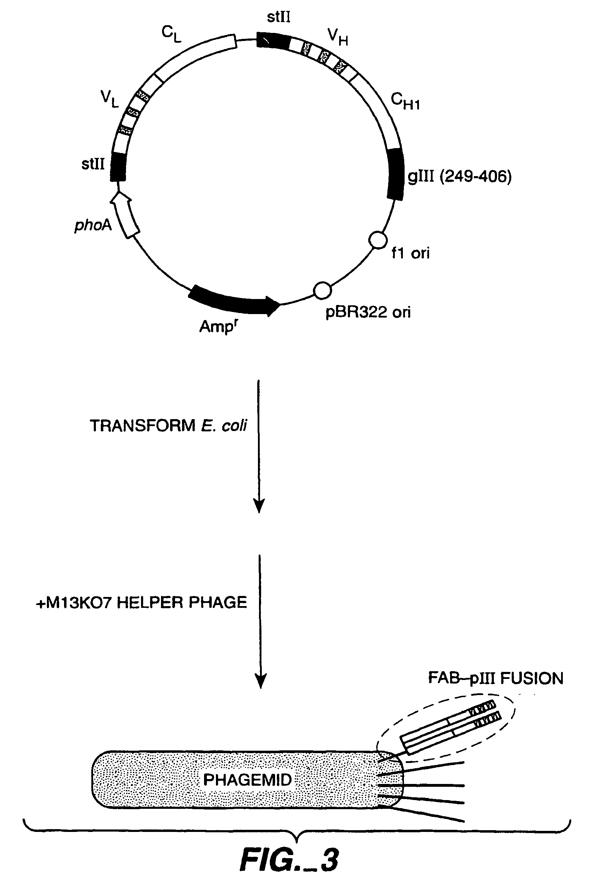

Figure 3 depicts the phagemid construct for surface display of Fab-pIII fusions on phage. The phagemid construct encodes a humanized version of the Fab fragment for antibody A4.6.1 fused to a portion of the M13 gene III coat protein. The fusion protein consists of the Fab joined at the carboxyl terminus of the heavy chain to a single glutamine residue (from suppression of an amber codon in supE *E. coli*), then the C-terminal region of the gene III protein (residues 249-406). Transformation into F⁺ *E. coli,* followed by superinfection with M13KO7 helper phage, produces phagemid particles in which a small proportion of these display a single copy of the fusion protein.

### Detailed Description of the Invention:

### A. Definitions

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one and (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains. Clothia et al., J. Mol. Biol. 186, 651 (1985); Novotny et al., PNAS USA 82, 4592 (1985).

The term "variable" refers to the fact that certain portions of the variable domains differ extensively in sequence among antibodies and are used in the binding and specificity of each particular antibody for its particular antigen. However, the variability is not evenly distributed through the variable domains of antibodies. It is concentrated in three segments called "complementarily determining regions" (CDRs) or "hypervariable regions" both in the light chain and the heavy chain variable domains. The more highly conserved portions of variable domains are called the framework (FR). The variable domains of native heavy and light chains each comprise four FR regions, largely adopting a p-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies. Kabat et al., supra. The constant domains are not involved directly in binding an antibody to an antigen, but exhibit various effector functions, such as participation of the antibody in antibody-dependent cellular toxicity.

Papain digestion of antibodies produces two identical antigen binding fragments, called Fab fragments, each with a single antigen binding site, and a residual "Fc" fragment, whose name reflects its ability to crystallize readily. Pepsin treatment yields an F(ab¹)₂ fragment that has two antigen combining sites and is still capable of cross linking antigen.

"Fv" is the minimum antibody fragment which contains a complete antigen recognition and binding site. This region consists of a dimer of one heavy and one light chain variable domain in tight, non-covalent association. It is in this configuration that the three CDRs of each variable domain interact to define an antigen binding site on the surface of the V_{H}-V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

A "Fab" fragment contains the constant domain of the light chain and the first constant domain (CHI) of the heavy chain. Fab¹ fragments differ from Fab fragments by the addition of a few residues at the carboxy terminus of the heavy chain CH1 domain including one or more cysteines from the antibody hinge region. Fab¹-SH is the designation herein for Fab¹ in which the cysteine residue(s) of the constant domains bear a free thiol group. F(ab¹)₂ antibody fragments originally were produced as pairs of Fab' fragments which have hinge cysteines between them. Other, chemical couplings of antibody fragments are also known.

The light chains of antibodies (immunoglobulins) from any vertebrate species can be assigned to one of two clearly distinct types, called kappa (κ)and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG and IgM, and several of these may be further divided into subclasses (isotypes), *e.g*. IgG-1, IgG-2, IgG-3, and IgG-4; IgA-1 and IgA-2. The heavy chain constant domains that correspond to the different classes of immunoglobulins are called α, delta, epsilon, γ, and, µ, respectively. The subunit structures and three-dimensional configurations of different classes of immunoglobulins are well known.

The term "antibody" is used in the broadest sense and specifically covers single monoclonal antibodies (including agonist and antagonist antibodies), antibody compositions with polyepitopic specificity, as well as antibody fragments (*e.g*., Fab, F(ab¹)₂, and Fv), so long as they exhibit the desired biological activity.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256, 495 (1975), or may be made by recombinant DNA methods, see, *e.g.* U.S. Patent No. 4,816,567.

"Chimeric" antibodies (immunoglobulins) are antibodies wherein a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. U.S. Patent No. 4,816,567.

"Humanized" forms of non-human (*e.g*. murine) antibodies are chimeric immunoglobulins, immunoglobulin chains or fragments thereof (such as Fv, Fab, Fab¹, F(ab¹ )₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin. For further details see: Jones et al., Nature 321, 522 (1986); Reichmann et al., Nature 332, 323 (1988); and Presta, Curr. Op. Struct. Biol. 2, 593 (1992).

"Non-immunogenic in a human" means that upon contacting the humanized antibody in a therapeutically effective amount with appropriate tissue of a human, a state of sensitivity or resistance to the humanized antibody is not substantially demonstratable upon administration.

As used herein, "vascular endothelial cell growth factor," or "VEGF," refers to a mammalian growth factor as defined in U.S. Patent 5,332,671, including the human amino acid sequence of Fig. 1. The biological activity of native VEGF is shared by any analogue or variant thereof that is capable of promoting selective growth of vascular endothelial cells but not of bovine corneal endothelial cells, lens epithelial cells, adrenal cortex cells, BHK-21 fibroblasts, or keratinocytes, or that possesses an immune epitope that is immunologically cross-reactive with an antibody raised against at least one epitope of the corresponding native VEGF.

"Site-directed mutagenesis" is a technique standard in the art, and is conducted using a synthetic oligonucleotide primer complementary to a single-stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells that harbor the phage. Theoretically, 50% of the new plaques will contain the phage having, as a single strand, the mutated form; 50% will have the original sequence. The plaques are hybridized with kinased synthetic primer at a temperature that permits hybridization of an exact match, but at which the mismatches with the original strand are sufficient to prevent hybridization. Plaques that hybridize with the probe are then selected and cultured, and the DNA is recovered.

"Expression system" refers to DNA sequences containing a desired coding sequence and control sequences in operable linkage, so that hosts transformed with these sequences are capable of producing the encoded proteins. To effect transformation, the expression system may be included on a vector; however, the relevant DNA may then also be integrated into the host chromosome.

As used herein, "cell," "cell line," and "cell culture" are used interchangeably and all such designations include progeny. Thus, "transformants" or "transformed cells" includes the primary subject cell and cultures derived therefrom without regard for the number of transfers. It is also understood that all progeny may not be precisely identical in DNA content, due to deliberate or inadvertent mutations. Mutant progeny that have the same functionality as screened for in the originally transformed cell are included. Where distinct designations are intended, it will be clear from the context.

"Plasmids" are designated by a lower case p preceded and/or followed by capital letters and/or numbers. The starting plasmids herein are commercially available, are publicly available on an unrestricted basis, or can be constructed from such available plasmids in accord with published procedures. In addition, other equivalent plasmids are known in the art and will be apparent to the ordinary artisan.

"Affinity binding" refers to the strength of the sum total of noncovalent interactions between a single antigen-binding site on an antibody and a single epitope. Low-affinity antibodies bind antigen weakly and tend to dissociate readily, whereas high-affinity antibodies bind antigen more tightly and remain bound longer.

"Transformation" means introducing DNA into an organism so that the DNA is replicable, either as an extrachromosomal element or by chromosomal integration. Depending on the host cell used, transformation is done using standard techniques appropriate to such cells. The calcium treatment employing calcium chloride, as described by Cohen, Proc. Natl. Acad Sci. USA 69, 2110 (1972) and Mandel et al., J. Mol. Biol. 53, 154 (1970), is generally used for prokaryotes or other cells that contain substantial cell-wall barriers. For mammalian cells without such cell walls, the calcium phosphate precipitation method of Graham and van der Eb, Virology 52, 456 (1978) is preferred. General aspects of mammalian cell host system transformations have been described by Axel in U.S. Pat. No. 4,399,216 issued August 16, 1983. Transformations into yeast are typically carried out according to the method of Van Solingen et al., J. Bact. 130, 946 (1977) and Hsiao et al., Proc. Natl. Acad. Sci. USA 76, 3829 (1979). However, other methods for introducing DNA into cells such as by nuclear injection, electroporation or by protoplast fusion may also be used.

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see Lawn et al., Nucleic Acids Res. 9, 6103 (1981) and Goeddel et al., Nucleic Acids Res. 8, 4057 (1980).

"Ligation" refers to the process of forming phosphodiester bonds between two double stranded nucleic acid fragments. Unless otherwise provided, ligation may be accomplished using known buffers and conditions with 10 units of T4 DNA ligase ("ligase") per 0.5 mg of approximately equimolar amounts of the DNA fragments to be ligated.

The term "control sequences" refers to DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancers.

Nucleic acid is "operably linked" or "operatively linked" when it is placed into a functional relationship with another nucleic acid sequence. For example, DNA for a presequence or a secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" or "operatively linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, then synthetic oligonucleotide adaptors or linkers are used in accord with conventional practice.

As used herein, "representatively numbered" refers to a position number of a residue in a particular sequence and corresponding position numbers in different sequences. Corresponding position numbers are those positions within sequences, generally human antibody framework sequences, which are functionally equivalent to the respresentatively numbered position when used in the construction of a humanized antibody.

Ordinarily, the terms "amino acid" and "amino acids" refer to all naturally occurring L-α-amino acids. In some embodiments, however, D-amino acids may be present in the polypeptides or peptides of the present invention in order to facilitate conformational restriction. The amino acids are identified by either the single-letter or three-letter designations:

| | | | | | |
|---|---|---|---|---|---|
| Asp | D | aspartic acid | Ile | I | isoleucine |
| Thr | T | threonine | Leu | L | leucine |
| Ser | S | serine | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |
| Val | V | valine | Gln | Q | glutamine |
| Met | M | methionine | Asn | N | asparagine |

The term "amino acid sequence variant" refers to molecules with some differences in their amino acid sequences as compared to a native amino acid sequence.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Hybridization is preferably performed under "stringent conditions" which means (1) employing low ionic strength and high temperature for washing, for example, 0.015 sodium chloride/0.0015 M sodium citrate/0.1% sodium dodecyl sulfate at 50°C, or (2) employing during hybridization a denaturing agent, such as formamide, for example, 50% (vol/vol) formamide with 0.1% bovine serum albumin/0.1% Ficoll/0.1% polyvinylpyrrolidone/50 nM sodium phosphate buffer at pH 6.5 with 750 mM sodium chloride, 75 mM sodium citrate at 42°C. Another example is use of 50% formamide, 5 x SSC (0.75 M NaCl, 0.075 M sodium citrate), 50 mM sodium phosphate (pH 6/8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC and 0.1% SDS. Yet another example is hybridization using a buffer of 10% dextran sulfate, 2 x SSC (sodium chloride/sodium citrate) and 50% formamide at 55°C, followed by a high-stringency wash consisting of 0.1 x SSC containing EDTA at 55°C. When a nucleic acid sequence of a nucleic acid molecule is provided, other nucleic acid molecules hybridizing thereto under the conditions described above are considered within the scope of the sequence.

Where amino acid sequences are described it is understood that these sequences can be reproduced by reconstructing the amino acid sequence synthetically or by mutation. Alternatively, it is understood that recombinant techniques can be used such that the DNA encoding the amino acid sequences is recovered. The DNA is recovered by forming a library from the DNA encoding the desired amino acid sequences. Probes are then generated based on the amino acid sequences. DNA hybridizing to the probes is then isolated and analyzed to determine whether the product encoded by the DNA is the desired product. Generally, cells are transformed with the DNA (or RNA) and expression studies are performed.

### B. General Methodology for Humanizing Antibodies

The methods described herein can be used to humanize any antibody. Similarly, it is understood that the humanized antibody specifically described herein, humanized anti-VEGF, can be reproduced by the methods described herein or by traditional DNA recombinant techniques. Specifically, since the critical framework residue mutations are described herein, the humanized antibody can be reproduced to have the same mutations without being reproduced using the monovalent phage display system. Rather, the DNA encoding the described amino acid sequences can be synthesized or reproduced by traditional DNA recombinant techniques. The DNA product can then be expressed, identified and recovered. Alternatively, site-directed mutagenesis can be performed on the antibody by methods known in the art, or the antibody can be synthesized so as to have the mutations described herein.

A particularly preferred method for producing the humanized antibodies described herein involves the following: preparing an antibody phagemid vector for monovalent display of Fab fragments having CDR sequences transplanted by site-directed mutagenesis onto a vector which codes for a human V_{L}κI-Cκ_{I} light chain and human V_{H} III-C_{H} lγ heavy chain Fd; constructing the antibody Fab phagemid library by random mutagenesis of a small set of selected critical framework residues; expressing and purifying the humanized Fab fragments; selecting humanized Fab variants; and, determining binding affinities. These steps do not have to be performed in any particular order. These steps are specifically described below in the "specific example" but are generally performed as follows:

### Preparation of antibody phagemid vector for monovalent display of Fab fragments

First an antibody to be humanized is selected and the complementary determining regions (CDRs) identified. The CDR sequences of the antibody can be identified according to the sequence definition of Kabat et al., supra. The CDR sequences are transplanted by site-directed mutagenesis onto a vector which codes for a human V_{L}κI-CκC_{I} light chain and human V_{H}III-C_{H}l_{γ} heavy chain Fd. The Fab encoding sequence can then be subcloned into a phagemid vector. This construct encodes an initial humanized antibody wherein the C-terminus of the heavy chain is fused precisely to the carboxyl portion of a phage coat protein. Perferably, a phagemid vector is selected which provides expression of both secreted heavy chain or heavy chain-gene III fusions in *supE* suppressor strains of *E. coli.*

### Construction of the antibody Fab phagemid library

Based on the cumulative results from humanizing a number of non-human antibodies onto a human V_{L}κI-V_{H}III framework, it was considered that framework changes required to optimize antigen binding are limited to some subset of the residues. See, Carter et al., PNAS USA 89, 4285 (1992); Presta et al., J. Immunol. 151, 2623 (1993); Eigenbrot et al., Proteins 18, 49-62 (1994); Shalaby et al., J. Exp. Med. 175, 217 (1992). Accordingly, a novel group of residues was selected for randomization. Randomizing these identified key framework residues provides the desired library of Fab variants to be displayed on the surface of filamentous phage. Specifically, V_{L} residues 4 and 71 and V_{H} residues 24, 37,67,69,71,71,75,76,78,93 and 94 have been selected as key framework residues important for antigen binding and targeted for randomization.

### Expression and purification of humanized Fab fragments

Various methods are known in the art to express and purify fragments. As described herein, an *E. coli* strain 34B8, a nonsuppressor, was transformed with phagemid pMB419, or variants thereof Single colonies were grown overnight at 37°C in 5 mL 2YT containing 50 µg/mL carbenicillin. These cultures were diluted into 200 mL AP5 medium, described in Chang et al., Gene 55, 189 (1987), containing 20 µg/mL carbenicillin and incubated for 26 hours at 30°C. The cells were pelleted at 4000 x g and frozen at -20°C for at least 2 hours. Cell pellets were then resuspended in 5 mL of 10 mM Tris-HCl (pH 7.6) containing 1 mM EDTA, shaken at 4 ° C for 90 minutes and centrifuged at 10,000 x g for 15 minutes. The supernatant was applied to a 1 mL streptococcal protein G-SEPHAROSE column (a column produced by Pharmacia) and washed with 10 mL of 10 mM MES (pH 5.5). The bound Fab fragment was eluted with 2.5 mL 100 mM acetic acid and immediately neutralized with 0.75 mL 1M TrisHCl, pH 8.0. Fab preparations were buffer-exchanged into PBS and concentrated using CENTRICON-30 concentrators (produced by Amicon). Typical yields ofFab were approximately 1 mg/L culture, post-protein G purification. Purified Fab samples were characterized by electrospray mass spectrometry, and concentrations were determined by amino acid analysis.

### Selection of humanized Fab variants

Purified labeled antigen is coated onto a microtiter plate. The coating solution is discarded, the wells blocked, and phagemid stock is added. After a period, the wells are washed and the bound phage eluted and titered. The remaining phage eluted from the VEGF-coated well are propagated for use in the next selection cycle. This process can be repeated several times to obtain the desired number of clones. For example, a few dozen individual clones can be selected and sequenced.

### Determination of VEGF binding affinities

Association and dissociation rate constants for binding of the humanized variants to VEGF are measured. Binding profiles are analyzed and those variants showing the highest affinities are selected.

### Administration of the humanized anti-VEGF

Administration of the humanized anti-VEGF can be extrapolated from the data presented on the murine anti-VEGF described in Kim et al., Growth Factors 7, 53 (1992); Kim et al., Nature 362, 841 (1993). In particular, Kim et al. demonstrates that as little as 10 µg twice weekly of the VEGF antibody resulted in significant inhibition of tumor growth. Maximal effects were achieved with antibody doses of 50-100 µg.

The following example is intended merely to illustrate the best mode now known for practicing the invention but the invention is not to be considered as limited to the details of this example.

### Specific Example I

### Construction of the phagemid vector and the initial humanized anti-VEGF

The murine anti-VEGF mAb A4.6.1 has been previously described by Kim et al, Growth Factors, 7, 53 (1992); Kim et al., Nature, 362, 841 (1993). The first Fab variant of humanized A4.6.1, hu2.0, was constructed by site-directed mutagenesis using a deoxyuridine-containing template of plasmid pAK2 which codes for a human V_{L}κI-Cκ_{I} light chain and human V_{H}III-C_{H} lγ₁ heavy chain Fd fragment. Carter et al., PNAS USA 89,4285 (1992). The transplanted A4.6.1 CDR sequences were chosen according to the sequence definition of Kabat et al., Sequences of Proteins of Immunological Interest (5th), Public Health Service, National Institutes of Health, Bethesda, MD. (1991), except for CDR-H1 which we extended to encompass both sequence and structural definitions, viz V_{H} residues 26-35, Chothia et al., J. Mol. Biol. 196, 901 (1987). The Fab encoding sequence was subcloned into the phagemid vector phGHamg3. Bass and Wells, Proteins, 8, 309 (1990); Lowman et al., Biochem. 30, 10832 (1991). This construct, pMB4-19, encodes the initial humanized A4.6.1 Fab, hu2.0, with the C-terminus of the heavy chain fused precisely to the carboxyl portion of the M13 gene III coat protein. pMB4-19 is similar in construction to pDH188, a previously described plasmid for monovalent display of Fab fragments. Garrard et al., Biotechn. 9: 1373-1377 (1991). Notable differences between pMB4-19 and pDH188 include a shorter M13 gene III segment (codons 249-406) and use of an amber stop codon immediately following the antibody heavy chain Fd fragment. This permits expression of both secreted heavy chain or heavy chain-gene III fusions in *supE* suppressor strains of *E. coli.*

The initial humanized A4.6.1 Fab fragment (hu2.0) in which the CDRs from A4.6.1 were grafted onto a human V_{Lκ}I-V_{H}III framework is shown in Figure 1. The V_{L} domain of hu2.0 is set forth in SEQ ID NO: 7 and the V_{H} domain of hu2.0 is set forth in SEQ ID NO: 10.

All residues other than the grafted CDRs were maintained as the human sequence. Binding of this initial humanized antibody to VEGF was so weak as to be undetectable. Based on the relative affinity of other weakly-binding humanized A4.6.1 variants (data not shown), the K_{D} for binding of hu2.0 was estimated at >7 µM. This contrasts with an affinity of 1.6 nM for a chimeric Fab construct consisting of the intact V_{L} and V_{H} domains from murine A4.6.1 and human constant domains. Thus, binding of hu2.0 to VEGF was at least 4000-fold reduced relative to the chimera.

### Design of the anti-VEGF Fab Phagemid library

The group of framework changes required to optimize antigen binding when using human V_{L}κI-V_{H}III framework were selected as shown in Table 1 and Figure 2. The humanized A4.6.1 phagemid library was constructed by site-directed mutagenesis according to the method of Kunkel et al., Methods Enzymol. 204, 125 (1991). A derivative of pMB4-19 containing TAA stop triplets at V_{H} codons 24, 37, 67 and 93 was prepared for use as the mutagenesis template (all sequence numbering according to Kabat et al., supra. This modification was to prevent subsequent background contamination by wild type sequences. The codons targeted for randomization were 4 and 71 (light chain) and 24, 37, 67, 69, 71, 73, 75, 76, 78, 93 and 94 (heavy chain).

**Table 1: Key framework residues important for antigen binding and targeted for randomization**

| Framework residue | | Human Vκ_{L}I, V_{H}III consensus residue | Murine A4.6.1 residue | Randomization^{a} |
|---|---|---|---|---|
| V_{L}: | 4 | Met | Met | Met,Leu |
| | 71 | Phe | Tyr | Phe, Tyr |
| | | | | |
| V_{H}: | 24 | Ala | Ala | Ala, Val, Thr |
| | 37 | Val | Val | Val, Ile |
| | 67 | Phe | Phe | Phe, Val, Thr, Leu, Ile, Ala |
| | 69 | Ile | Phe | Ile, Phe |
| | 71 | Arg | Leu | Arg^{b}, Leu^{b} |
| | 73 | Asp | Thr | Asp^{b}, Thr^{b} |
| | 75 | Lys | Ala | Lys^{b}, Ala^{b} |
| | 76 | Asn | Ser | Asn^{b}, Ser^{b} |
| | 78 | Leu | Ala | Leu, Ala, Val, |
| Phe | | | | |
| | 93 | Ala | Ala | Ala, Val, Leu, |
| Ser, | | | | Thr |
| | | Arg | Lys | Arg, Lys |

| | | | | |
|---|---|---|---|---|
| ^{a}Amino acid diversity in phagemid library ^{b} V_{H} 71, 73, 75, 76 randomized to yield the all-murine (L71/T73/A75/S76) or all-human (R71/D73/K75/N76) V_{H}III tetrad | | | | |

A concern in designing the humanized A4.6.1 phagemid library was that residues targeted for randomization were widely distributed across the V_{L} and V_{H} sequences. Limitations in the length of synthetic oligonucleotides requires that simultaneous randomization of each of these framework positions can only be achieved through the use of multiple oligonucleotides. However, as the total number of oligonucleotides increases, the efficiency of mutagenesis decreases (*i.e*. the proportion of mutants obtained which incorporate sequence derived from all of the mutagenic oligonucleotides). To circumvent this problem, two features were incorporated into the library construction. The first was to prepare four different mutagenesis templates coding for each of the possible V_{L} framework combinations. This was simple to do given the limited diversity of the light chain framework (only 4 different sequences), but was beneficial in that it eliminated the need for two oligonucleotides from the mutagenesis strategy. Secondly, two 126 base oligonucleotides were preassembled from smaller synthetic fragments. This made possible randomization of V_{H} codons 67, 69, 71, 73, 75, 76, 93 and 94 with a single long oligonucleotide, rather than two smaller ones. The final randomization mutagenesis strategy therefore employed only two oligonucleotides simultaneously onto four different templates.

More specifically, in order to randomize heavy chain codons 67, 69, 71, 73, 75, 76, 78, 93 and 94 with a single mutagenic oligonucleotide, two 126-mer oligonucleotides were first preassembled from 60 and 66-mer fragments by template-assisted enzymatic ligation. Specifically, 1.5 nmol of 5' phosphorylated oligonucleotide GAT TTC AAA CGT CGT NYI ACT WTT TCT AGA GAC AAC TCC AAA AAC ACA BYT TAC CTG CAG ATG AAC (SEQ ID NO: 12) or GAT TTC AAA CGT CGT NYT ACT WTT TCT TTA GAC ACC TCC GCA AGC ACA BYT TAC CTG CAG ATG AAC (SEQ ID NO: 1) were combined with 1.5 nmol of AGC CTG CGC GCT GAG GAC ACT GCC GTC TAT TAC TGT DYA ARG TAC CCC CAC TAT TAT GGG (SEQ ID NO: 2). The randomized codons are underlined and N represents A/G/T/C; W represents A/T; B represents G/T/C; D represents G/A/T; R represents A/G; and Y represents C/T ("/" represents "or"). Then, 1.5 nmol of template oligonucleotide CTC AGC GCG CAG GCT GTT CAT CTG CAG GTA (SEQ ID NO: 3), with complementary sequence to the 5' ends of SEQ ID NOS: 12 and I and the 3' end of SEQ ID NO: 3 was added to hybridize to each end of the ligation junction. To this mixture, *Taq* ligase (thermostable ligase from New England Biolabs) and buffer were added, and the reaction mixture was subjected to 40 rounds of thermal cycling, (95°C for 1.25 minutes and 50°C for 5 minutes) so as to cycle the template oligonucleotide between ligated and unligated junctions. The product 126-mer oligonucleotides were purified on a 6% urea/TBE polyacrylamide gel and extracted from the polyacrylamide in buffer. The two 126-mer products were combined in equal ratio, ethanol precipitated and finally solubilized in 10 mM Tris-HCl, 1 mM EDTA. The mixed 126-mer oligonucleotide product was labeled 504-01.

Randomization of select framework codons (V_{L} 4, 71; V_{H} 24, 37, 67, 69, 71, 73, 75, 76, 93, 94) was thus effected in two steps. First, V_{L} randomization was achieved by preparing three additional derivatives of the modified pMB4-19 template. Framework codons 4 and 71 in the light chain were replaced individually or pairwise using the two mutagenic oligonucleotides GCT GAT ATC CAG TTG ACC CAG TCC CCG (SEQ ID NO: 13) and TCT GGG ACG GAT TAC ACT CTG ACC ATC (SEQ ID NO: 4). Deoxyuridine containing template was prepared from each of these new derivatives. Together with the original template, these four constructs coded for each of the four possible light chain framework sequence combinations (see Table 1).

Oligonucleotides 504-01, the mixture of two 126-mer oligonucleotides, and CGT TTG TCC TGT GCA RYT TCT GGC TAT ACC TTC ACC AAC TAT GGT ATG AAC TGG RTC CGT CAG GCC CCG GGT AAG (SEQ ID NO: 5) were used to randomize heavy chain framework codons using each of the four templates just described. The four libraries were electroporated into *E. coli* XL-1 BLUE CELLS (marker cells produced by Stratagene) and combined. The total number of independent transformants was estimated at >1.2 x 10⁸, approximately 1,500-fold greater than the maximum number of DNA sequences in the library.

From this strategy, each of residues 4 and 71 in the light chain and 24, 37, 67, 78 and 93 from the heavy chain were partially randomized to allow the selection of either the murine A4.6.1, human V_{L}κI-V_{H}III sequence, or sequences commonly found in other human and murine frameworks (Table I). Note that randomization of these residues was not confined to a choice between the human V_{L}κI-V_{H}III consensus or A4.6.1 framework sequences. Rather, inclusion of additional amino acids commonly found in other human and murine framework sequences allows for the possibility that additional diversity may lead to the selection of tighter binding variants.

Some of the heavy chain framework residues were randomized in a binary fashion according to the human V_{H}III and murine A4.6.1 framework sequences. Residues V_{H} 71, 73, 75 and 76 are positioned in a hairpin loop adjacent to the antigen binding site. The side chains of V_{H} 71 and 73 are largely buried in canonical antibody structures and their potential role in shaping the conformation of CDR-H2 and CDR-H3 is well known. Kettleborough et al., Protein Eng. 4, 773 (1991); Carter et al., PNAS USA 89, 4285 (1992); Shalaby et al., J. Exp. Med. 175, 217 (1992). On the other hand, although the side chains of V_{H} 75 and 76 are solvent exposed (Figure 2), it has nevertheless been observed that these two residues can also influence antigen binding (Eigenbrot, Proteins 18, 49 [1994]), presumably due to direct antigen contact in some antibody-antigen complexes. Because of their proximity in sequence and possible interdependence, V_{H} 71, 73, 75 and 76 were randomized en bloc such that only two possible combinations of this tetrad could be selected; either all human V_{H}III or all murine A4.6.1 sequence. Finally, V_{H} residues 69 and 94 were randomized, but only to represent the V_{H}III and A4.6.1 sequences. The V_{H} 69 and 94 were not replaced in previous antibody humanizations, but because they differ between the V_{H}III consensus and A4.6.1 sequences (Figure 1) and have been noted as potentially important for proper CDR conformation (Foote et al., J. Mol. Biol. 224, 487 [1992]), they were included in this randomization strategy.

### Humanized A4.6.1 Fab library displayed on the surface of phagemid

A variety of systems have been developed for the functional display of antibody fragments on the surface of filamentous phage. Winter et al., Ann. Rev. Immunol. 12, 433 (1994). These include the display of Fab or single chain Fv (scFv) fragments as fusions to either the gene III or gene VIII coat proteins of M13 bacteriophage. The system selected herein is similar to that described by Garrard et al., Biotechn. 9, 1373 (1991) in which a Fab fragment is monovalently displayed as a gene III fusion (Figure 3). This system has two notable features. In particular, unlike scFvs, Fab fragments have no tendency to form dimeric species, the presence of which can prevent selection of the tightest binders due to avidity effects. Additionally, the monovalency of the displayed protein eliminates a second potential source of avidity effects that would otherwise result from the presence of multiple copies of a protein on each phagemid particle. Bass and Wells, Proteins 8, 309 (1990); Lowman et al., Biochemistry 30, 10832 (1991).

Phagemid particles displaying the humanized A4.6.1 Fab fragments were propagated in *E. coli* XL-1 Blue cells. Briefly, cells harboring the randomized pMB4-19 construct were grown overnight at 37°C in 25 mL 2YT medium containing 50 µg/mL carbenicillin and approximately 10¹⁰ M13KO7 helper phage (Viera and Messing, Methods Enzymol. 153, [1987]). Phagemid stocks were purified from culture supernatants by precipitation with a saline polyethylene glycol solution, and resuspended in 100 µL PBS (approximately 10¹⁴ phagemid/mL).

### Selection of humanized A4.6.1 Fab variants

Purified VEGF₁₂₁ (100 µL at 10 µg/mL in PBS) was coated onto a microtiter plate well overnight at *4°C. The coating solution was discarded and this well and an uncoated well were blocked with 6% skim milk for 1 hour and washed with PBS containing 0.05% TWEEN-20 (detergent). Then, 10 µL of phagemid stock, diluted to 100 µL with 20 mM Tris (pH 7.5) containing 0.1% BSA and 0.05% TWEEN-20, was added to each well. After 2 hours, the wells were washed and the bound phage eluted with 100 µL of 0.1 M glycine (pH 2.0), and neutralized with 25 µL of 1M Tris pH 8.0. An aliquot of this was used to titer the number of phage eluted. The remaining phage eluted from the VEGF-coated well were propagated for use in the next selection cycle. A total of 8 rounds of selection was performed after which time 20 individual clones were selected and sequenced (Sanger et al., PNAS USA 74, 5463 [1977]).

Variants from the humanized A4.6.1 Fab phagemid library were thusly selected based on binding to VEGF. Enrichment of functional phagemid, as measured by comparing titers for phage eluted from a VEGF-coated versus uncoated microtiter plate well, increased up to the seventh round of affinity panning. After one additional round of sorting, 20 clones were sequenced to identify preferred framework residues selected at each position randomized. These results, summarized in Table 2, revealed strong consensus amongst the clones selected. Ten out of the twenty clones had the identical DNA sequence, designated hu2.10. Of the thirteen framework positions randomized, eight substitutions were selected in hu2.10 (V_{L} 71; V_{H} 37, 71, 73, 75, 76, 78 and 94). Interestingly, residues VH 37 (Ile) and 78 (Val) were selected neither as the human V_{H}III or murine A4.6.1 sequence. This result suggests that some framework positions may benefit from extending the diversity beyond the target human and parent murine framework sequences.

**Table 2: Sequences selected from the humanized A4.6.1 phagemid Fab library**

| Variant | Residue substitutions | | | | | | | | | | | | |
|---|---|---|---|---|---|---|---|---|---|---|---|---|---|
| | V_{L} | | V_{H} | | | | | | | | | | |
| | 4 | 71 | 24 | 37 | 67 | 69 | 71 | 73 | 75 | 76 | 78 | 93 | 94 |
| murine A4.6.1 | M | Y | A | V | F | F | L | T | A | S | A | A | K |
| hu2.0 (CDR-graft) | M | F | A | V | F | I | R | N | K | N | L | A | R |
| **Phage-selected clones:** | | | | | | | | | | | | | |
| hu2.1 (2) | - | Y | - | I | - | - | - | - | - | - | V | - | K |
| hu2.2 (2) | L | Y | - | I | - | - | - | - | - | - | V | - | K |
| hu2.6 (1) | L | - | - | I | | T - | L | T | A | S | V | - | K |
| hu2.7 (1) | L | - | - | I | T | - | - | - | - | - | V | - | K |
| hu2.10 (10) | - | Y | - | I | - | - | L | T | A | S | V | - | K |

| | | | | | | | | | | | | | |
|---|---|---|---|---|---|---|---|---|---|---|---|---|---|
| Differences between hu2.0 and murine A4.6.1 antibodies are underlined. The number of identical clones identified for each phage-selected sequence is indicated in parentheses. Dashes in the sequences of phage-selected clones indicate selection of the human V_{L}κI-V_{H}III framework sequence (i.e. as in hu2.0). | | | | | | | | | | | | | |

There were four other unique amino acid sequences among the remaining ten clones analyzed: hu2.1, hu2.2, hu2.6 and hu2.7. All of these clones, in addition to hu2.10, contained identical framework substitutions at positions V_{H} 37 (Ile), 78 (Val) and 94 (Lys), but retained the human V_{H}III consensus sequence at positions 24 and 93. Four clones had lost the light chain coding sequence and did not bind VEGF when tested in a phage ELISA assay (Cunningham et al., EMBO J. 13, 2508 [1994]). We have occasionally noted the loss of heavy or light chain sequence with other Fab phagemid libraries (unpublished data), and these clones are presumably selected for on the basis of enhanced expression. Such artifacts can often be minimized by reducing the number of sorting cycles or by propagating libraries on solid media.

### Determination of VEGF binding affinities

Association (kₒₙ) and dissociation (k_{off}) rate constants for binding of humanized A4.6.1 Fab variants to VEGF₁₂₁ were measured by surface plasmon resonance (Karlsson et al, J. Immun. Methods 145, 229 [1991]) on a Pharmacia BIAcore instrument. VEGF₁₂₁ was covalently immobilized on the biosensor chip via primary amino groups. Binding of humanized A4.6.1 Fab variants was measured by flowing solutions of Fab in PBS/0.05% TWEEN-20 (detergent) over the chip at a flow rate of 20 µL/min. Following each binding measurement, residual Fab was stripped from the immobilized ligand by washing with 5 µL of 50 mM aqueous HCl at 3 µL/min. Binding profiles were analyzed by nonlinear regression using a simple monovalent binding model (BIAevaluation software v2.0; Pharmacia).

Phage-selected variants hu2.1, hu2.2, hu2.6, hu2.7 and hu2.10 were expressed in *E. coli* using shake flasks and Fab fragments were purified from periplasmic extracts by protein G affinity chromatography. Recovered yields of Fab for these five clones ranged from 0.2 (hu2.6) to 1.7 mg/L (hu2.1). The affinity of each of these variants for antigen (VEGF) measured by surface plasmon resonance on a BIAcore instrument as shown in Table 3.

**Table 3: VEGF binding affinity of humanized A4.6.1 Fab variants.**

| Variant | kₒₙ | k_{off} | K_{D} | K_{D-(A4,6,1)} K_{D} (mut) |
|---|---|---|---|---|
| | M⁻¹s⁻¹/10⁴ | 10⁴s⁻¹ | nM | |
| A4.6.1 chimera | 5.4 | 0.85 | 1.6 | |
| hu2.0 | ND | ND | >7000** | >4000 |
| **Phage selected clones:** | | | | |
| hu2.1 | 0.70 | 18 | 260 | 170 |
| hu2.2 | 0.47 | 16 | 340 | 210 |
| hu2.6 | 0.67 | 4. 5 | 67 | 40 |
| hu2.7 | 0.67 | 24 | 360 | 230 |
| hu2.10 | 0.63 | 3.5 | 55 | 35 |
| *hu2.10V | 2.0 | 1.8 | 9.3 | 5.8 |

| | | | | |
|---|---|---|---|---|
| *hu2.10V = hu2.10 with mutation V_{L} Leu46 -> Val; Estimated errors in the Biacore binding measurements are +/- 25%; **Too weak to measure, estimate of lower bound | | | | |

Analysis of this binding data revealed that the consensus clone hu2.10 possessed the highest affinity for VEGF out of the five variants tested. Thus our Fab phagemid library was selectively enriched for the tightest binding clone. The calculated K_{D} for hu2.10 was 55 nM, at least 125-fold tighter than for hu2.0- which contains no framework changes (K_{D} >7 µM). The other four selected variants all exhibited weaker binding to VEGF, ranging down to a K_{D} of 360 nM for the weakest (hu2.7). Interestingly, the K_{D} for hu2.6, 67 nM, was only marginally weaker than that of hu2.10 and yet only one copy of this clone was found among 20 clones sequenced. This may have due to a lower level of expression and display, as was the case when expressing the soluble Fab of this variant. However, despite the lower expression rate, this variant is useful as a humanized antibody.

### Additional improvement of humanized variant hu2, 10

Despite the large improvement in antigen affinity over the initial humanized variant, binding of hu2.10 to VEGF was still 35-fold weaker than a chimeric Fab fragment containing the murine A4.6.1 V_{L} and V_{H} domains. This considerable difference suggested that further optimization of the humanized framework might be possible through additional mutations. Of the vernier residues identified by Foote et al., J. Mol. Biol. 196, 901 (1992), only residues V_{L} 46, V_{H} 2 and V_{H} 48 differed in the A4.6.1 versus human V_{I}κI-V_{H}III framework (Figure 1) but were not randomized in our phagemid library. A molecular model of the humanized A4.6.1 Fv fragment showed that V_{L} 46 sits at the V_{L}-V_{H} interface and could influence the conformation of CDRH3. Furthermore, this amino acid is almost always leucine in most V_{L}κ frameworks (Kabat et al., supra.), but is valine in A4.6.1. Accordingly, a Leu -> Val substitution was made at this position in the background of hu2.10. Analysis of binding kinetics for this new variant, hu2. 10V, indicated a further 6-fold improvement in the K_{D} for VEGF binding. The K_{D} for hu2. 10V (9.3 nM) was thus within 6-fold that of the chimera. In contrast to V_{L} 46, no improvement in the binding affinity of hu2.10 was observed for replacement of either V_{H} 2 or V_{H} 48 with the corresponding residue from murine A4.6.1.

Interestingly, part of the improvement prior to the last change in affinity was due to an increase in the association rate constant (kₒₙ), suggesting that V_{L} 46 may play a role in preorganizing the antibody structure into a conformation more suitable for antigen binding. Other mutations which affected antigen affinity were primarily due to changes in the dissociation rate constant (k_{off}) for binding. Comparison of hu2.1 and hu2.10 reveals a 5-fold improvement in affinity for substitution of V_{H} residues 71, 73, 75, 76 with the A4.6.1 sequence. Conversion of V_{L}- 71 to the A4.6.1 sequence (Phe -> Tyr) had negligible effect on binding (hu2.2 vs hu2.7), while variants with leucine at V_{L} 4 bound marginally worse (<2-fold) than those with methionine, the naturally occurring residue in both the A4.6. 1 and human V_{KL}I frameworks (hu2.2 vs hu2.1). Comparison of other humanized A4.6.1 variants not shown here revealed that the V_{H} 94 Arg -> Lys change resulted in a 5-fold improvement in K_{D}, either due to direct antigen contact by this residue, or to a structural role in maintaining the proper conformation of CDR-H3. Variant hu2.6 has three sequence differences relative to the consensus clone hu2.10, but nevertheless has a similar K_{D}, thereby suggesting that these three substitutions have little effect on antigen binding. The negligible effect of conservative changes at V_{L} 4 and 71 concurs with binding data for other variants, yet the change at V_{H} 67 (Phe -> Thr) had little effect on binding.

### Concluding Remarks

The foregoing description details specific methods which can be employed to practice the present invention. Having detailed such specific methods, those skilled in the art will well enough know how to devise alternative reliable methods at arriving at the same information by using the fruits of the present invention. Thus, however detailed the foregoing may appear in text, it should not be construed as limiting the overall scope thereof.

## Claims

1. A method for producing and identifying framework mutations which improve the binding of humanised antibodies to their cognate antigens, the method comprising:
(a) grafting non-human complementarity determining regions (CDRs) onto a human framework comprising the V_{L}κ subgroup I (V_{L}κI) and V_{H} subgroup III (V_{H}III);
(b) randomly mutagenising framework residues of a set of thirteen critical framework positions;
(c) monovalently displaying the resultant library of antibody molecules on the surface of filamentous phage;
(d) identifying the optimal framework sequences by affinity-based selection; and
(e) optionally further mutating selected antibodies by replacing vernier residues which sit at the V_{L}-V_{H} interface with residues which match the non-human parent antibody,
wherein the critical framework residues of step (b) are V_{L} residues 4 and 71 and V_{H} residues 24, 37, 67, 69, 71, 73, 75, 76, 78, 93 and 94 (Kabat numbering).

2. The method of claim 1, wherein the critical framework residues are partially randomized to allow the selection of either a murine framework sequence, the human V_{L}κI-V_{H}III consensus framework sequence, or framework sequences commonly found in other human and murine frameworks.

3. The method of any one of claims 1 to 2, wherein the humanised antibody is an anti-VEGF antibody.

## Patentansprüche

1. Verfahren zum Herstellen und Identifizieren von Gerüstmutationen ("framework mutations"), die die Bindung von humanisierten Antikörpern an ihre entsprechenden Antigene verbessern, wobei das Verfahren die folgenden Schritte umfasst:
(a) Aufpfropfen von nicht-menschlichen komplementaritätsbestimmenden Regionen (complementarity determining regions; CDRs) auf ein menschliches Gerüst, das die V_{L}κ Untergruppe I (V_{L}κI) und V_{H} Untergruppe III (V_{H}III) umfasst;
(b) zufälliges Mutagenisieren von Gerüstresten eines Satzes von dreizehn kritischen Gerüstpositionen;
(c) monovalentes Präsentieren der resultierenden Bibliothek von Antikörpermolekülen auf der Oberfläche von filamentösen Phagen;
(d) Identifizieren der optimalen Gerüstsequenzen durch Affinitäts-basierte Selektion; und
(e) gegebenenfalls weiteres Mutieren ausgewählter Antikörper durch Austauschen von Feineinstellungsresten ("vernier residues"), die an der V_{L}-V_{H} Schnittstelle sitzen mit Resten, die dem nicht-menschlichen Ausgangsantikörper entsprechen,
wobei die kritischen Gerüstreste von Schritt (b) die V_{L} Reste 4 und 71 und die V_{H} Reste 24, 37, 67, 69, 71, 73, 75, 76, 78, 93 und 94 (Kabat Nummerierung) sind.

2. Verfahren nach Anspruch 1, wobei die kritischen Gerüstreste teilweise randomisiert sind, um die Selektion auf entweder eine murine Gerüstsequenz, der humanen V_{L}κI-V_{H}III Konsensus-Gerüstsequenz oder auf Gerüstsequenzen zu erlauben, die gewöhnlich in anderen humanen oder murinen Gerüsten gefunden werden.

3. Verfahren nach einem beliebigen der Ansprüche 1 bis 2, wobei der humanisierte Antikörper ein anti-VEGF Antikörper ist.

## Revendications

1. Procédé de production et d'identification des mutations de cadre qui améliorent la liaison des anticorps humanisés à leurs antigènes apparentés, le procédé comprenant :
(a) le greffage de régions déterminant la complémentarité non-humaines (les CDR) sur un cadre humain comprenant le sous-groupe I de V_{L}κ (V_{L}κI) et le sous-groupe III de V_{H} (V_{H}III) ;
(b) la mutagenèse aléatoire de résidus de cadre d'un ensemble de treize positions de cadre critiques ;
(c) l'exposition de manière monovalente de la banque de molécules d'anticorps résultante à la surface d'un phage filamenteux ;
(d) l'identification des séquences de cadre optimales par une sélection par affinité ; et
(e) éventuellement une mutation supplémentaire d'anticorps sélectionnés par le remplacement de résidus vernier qui siègent à l'interface V_{L}-V_{H} avec des résidus qui correspondent à l'anticorps parent non-humain,
dans lequel les résidus de cadre critiques de l'étape (b) sont des résidus de V_{L} 4 et 71 et des résidus de V_{H} 24, 37, 67, 69, 71, 73, 75, 76, 78, 93 et 94 (numérotation de Kabat).

2. Procédé selon la revendication 1, dans lequel les résidus de cadre critiques sont partiellement randomisés pour permettre la sélection soit d'une séquence de cadre murine, la séquence de cadre consensus V_{L}κI-V_{H}III humaine, soit des séquences de cadre généralement trouvées dans d'autres cadres humains et murins.

3. Procédé selon l'une quelconque des revendications 1 à 2, dans lequel l'anticorps humanisé est un anticorps anti-VEGF.


### SEQUENCE LISTING

(1) GENERAL INFORMATION:
   (i) APPLICANT: Genentech, Inc.
   (ii) TITLE OF INVENTION: HUMANIZED ANTIBODIES AND METHODS FOR FORMING HUMANIZED ANTIBODIES
   (iii) NUMBER OF SEQUENCES: 14
   (iv) CORRESPONDENCE ADDRESS:
      (A) ADDRESSEE: Flehr, Hohbach, Test, Albritton & Herbert
      (B) STREET: Four Embarcadero Center, Suite 3400
      (C) CITY: San Francisco
      (D) STATE: California
      (E) COUNTRY: United States
      (F) ZIP: 94111
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: PCT HEREWITH
      (B) FILING DATE: 02-APR-1998
      (C) CLASSIFICATION:
   (vii) PRIOR APPLICATION DATA:
      (A) APPLICATION NUMBER:08/833,504
      (B) FILING DATE: 07-APR-1997
   (viii) ATTORNEY/AGENT INFORMATION:
      (A) NAME: Dreger, Walter H.
      (B) REGISTRATION NUMBER: 24,190
      (C) REFERENCE/DOCKET NUMBER: A-64254
   (ix) TELECOMMUNICATION INFORMATION:
      (A) TELEPHONE: (415) 781-1989
      (B) TELEFAX: (415) 398-3249
(2) INFORMATION FOR SEQ ID N0:1:
   (i) SEQUENCE CHARACTERISTICS:
      (A) LENGTH: 66 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 60 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:2:
      AGCCTGCGCG CTGAGGACAC TGCCGTCTAT TACTGTDYAA RGTACCCCCA CTATTATGGG 60
(2) INFORMATION FOR SEQ ID NO:3:
   (i) SEQUENCE CHARACTERISTICS:
      (A) LENGTH: 30 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:3:
      CTCAGCGCGC AGGCTGTTCA TCTGCAGGTA 30
(2) INFORMATION FOR SEQ ID NO:4:
   (i) SEQUENCE CHARACTERISTICS:
      (A) LENGTH: 27 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:4:
      TCTGGGACGG ATTACACTCT GACCATC 27
(2) INFORMATION FOR SEQ ID **NO:5:**
   (i) SEQUENCE CHARACTERISTICS:
      (A) LENGTH: 75 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 107 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:6:
   Asp Ile Gln Met Thr Gln Thr Thr Ser Ser Leu Ser Ala Ser Leu Gly
   1               5                   10                  15
   Asp Arg Val Ile Ile Ser Cys Ser Ala Ser Gln Asp Ile Ser Asn Tyr
               20                  25                  30
   Leu Asn Trp Tyr Gln Gln Lys Pro Asp Gly Thr Val Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Thr Val Pro Trp
                   85                  90                  95
   Thr Phe Gly Gly Gly Thr Lys Leu Glu Ile Lys
               100                 105
(2) INFORMATION FOR SEQ ID NO:7:
   (i) SEQUENCE CHARACTERISTICS:
      (A) LENGTH: 107 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:7:
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys
               100                 105
(2) INFORMATION FOR SEQ ID NO:8:
   (i) SEQUENCE CHARACTERISTICS:
      (A) LENGTH: 107 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:8:
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys
               100                 105
(2) INFORMATION FOR SEQ ID NO:9:
   (i) SEQUENCE CHARACTERISTICS:
      (A) LENGTH: 123 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:9:
   Glu Ile Gln Leu Val Gln Ser Gly Pro Glu Leu Lys Gln Pro Gly Glu
   1               5                   10                  15
   Thr Val Arg Ile Ser Cys Lys Ala Ser Gly Tyr Thr Phe Thr Asn Tyr
               20                  25                  30
   Gly Met Asn Trp Val Lys Gln Ala Pro Gly Lys Gly Leu Lys Trp Met
           35                  40                  45
   Gly Trp Ile Asn Thr Tyr Thr Gly Glu Pro Thr Tyr Ala Ala Asp Phe
       50                  55                  60
   Lys Arg Arg Phe Thr Phe Ser Leu Glu Thr Ser Ala Ser Thr Ala Tyr
   65                  70                  75                  80
   Leu Gln Ile Ser Asn Leu Lys Asn Asp Asp Thr Ala Thr Tyr Phe Cys
                   85                  90                  95
   Ala Lys Tyr Pro His Tyr Tyr Gly Ser Ser His Trp Tyr Phe Asp Val
               100                 105                 110
   Trp Gly Ala Gly Thr Thr Val Thr Val Ser Ser
           115                 120
(2) INFORMATION FOR SEQ ID NO:10:
   (i) SEQUENCE CHARACTERISTICS:
      (A) LENGTH: 123 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:10:
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Thr Asn Tyr
               20                  25                  30
   Gly Met Asn Trp Val Arg Gln Ala Pro Gly Lys Gly Leu Glu Trp Val
           35                  40                  45
   Gly Trp Ile Asn Thr Tyr Thr Gly Glu Pro Thr Tyr Ala Ala Asp Phe
       50                  55                  60
   Lys Arg Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Pro His Tyr Tyr Gly Ser Ser His Trp Tyr Phe Asp Val
               100                 105                 110
   Trp Gly Gln Gly Thr Leu Val Thr Val Ser Ser
           115                 120
(2) INFORMATION FOR SEQ ID NO:11:
   (i) SEQUENCE CHARACTERISTICS:
      (A) LENGTH: 123 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:11:
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Thr Asn Tyr
               20                  25                  30
   Gly Met Asn Trp Ile Arg Gln Ala Pro Gly Lys Gly Leu Glu Trp Val
           35                  40                  45
   Gly Trp Ile Asn Thr Tyr Thr Gly Glu Pro Thr Tyr Ala Ala Asp Phe
       50                  55                  60
   Lys Arg Arg Phe Thr Ile Ser Leu Asp Thr Ser Ala Ser Thr Val Tyr
   65                  70                  75                  80
   Leu Gln Met Asn Ser Leu Arg Ala Glu Asp Thr Ala Val Tyr Tyr Cys
                   85                  90                  95
   Ala Lys Tyr Pro His Tyr Tyr Gly Ser Ser His Trp Tyr Phe Asp Val
               100                 105                 110
   Trp Gly Gln Gly Thr Ser Val Thr Val Ser Ser
           115                 120
(2) INFORMATION FOR SEQ ID NO:12:
   (i) SEQUENCE CHARACTERISTICS:
      (A) LENGTH: 66 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:12:
   GATTTCAAAC GTCGTNYTAC TWTTTCTAGA GACAACTCCA AAAACACABY TTACCTGCAG   60
   ATGAAC                                                              66
(2) INFORMATION FOR SEQ ID NO:13:
   (i) SEQUENCE CHARACTERISTICS:
      (A) LENGTH: 27 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:13:
      GCTGATATCC AGTTGACCCA GTCCCCG 27
(2) INFORMATION FOR SEQ ID NO:14:
   (i) SEQUENCE CHARACTERISTICS:
      (A) LENGTH: 6072 base pairs
      (B) TYPE: nucleic acid
      (C) STRANDEDNESS: unknown
      (D) TOPOLOGY: unknown
   (ii) MOLECULE TYPE: DNA (genomic)
   (ix) FEATURE:
      (A) NAME/KEY: misc feature
      (B) LOCATION: 459..460
      (D) OTHER INFORMATION: /note= "Light chain begins at base no. 959."
   (ix) FEATURE:
      (A) NAME/KEY: misc feature
      (B) LOCATION: 1101..1102
      (D) OTHER INFORMATION: /note= "Light chain terminates at base no. 1101."
   (ix) FEATURE:
      (A) NAME/KEY: misc feature
      (B) LOCATION: 1254..1255
      (D) OTHER INFORMATION: /note= "Heavy chain begins at base no. 1254."
   (ix) FEATURE:
      (A) NAME/KEY: misc_feature
      (B) LOCATION: 2424..2425
      (D) OTHER INFORMATION: /note= "Heavy chain terminates at base no. 2929."
   (xi) SEQUENCE DESCRIPTION: SEQ ID NO:14:
   GAATTCAACT TCTCCATACT TTGGATAAGG AAATACAGAC ATGAAAAATC TCATTGCTGA   60
   GTTGTTATTT AAGCTTTGGA GATTATCGTC ACTGCAATGC TTCGCAATAT GGCGCAAAAT  120
   GACCAACAGC GGTTGATTGA TCAGGTAGAG GGGGCGCTGT ACGAGGTAAA GCCCGATGCC  180
   AGCATTCCTG ACGACGATAC GGAGCTGCTG CGCGATTACG TAAAGAAGTT ATTGAAGCAT  240
   CCTCGTCAGT AAAAAGTTAA TCTTTTCAAC AGCTGTCATA AAGTTGTCAC GGCCGAGACT  300
   TATAGTCGCT TTGTTTTTAT TTTTTAATGT ATTTGTAACT AGAATTCGAG CTCGGTACCC  360
   GGGGATCCTC TAGAGGTTGA GGTGATTTTA TGAAAAAGAA TATCGCATTT CTTCTTGCAT  420
   CTATGTTCGT TTTTTCTATT GCTACAAACG CGTACGCTGA TATCCAGATG ACCCAGTCCC  480
   CGAGCTCCCT GTCCGCCTCT GTGGGCGATA GGGTCACCAT CACCTGCAGC GCAAGTCAGG  540
   ATATTAGCAA CTATTTAAAC TGGTATCAAC AGAAACCAGG AAAAGCTCCG AAAGTACTGA  600
   TTTACTTCAC CTCCTCTCTC CACTCTGGAG TCCCTTCTCG CTTCTCTGGA TCCGGTTCTG  660
   GGACGGATTA CACTCTGACC ATCAGCAGTC TGCAGCCAGA AGACTTCGCA ACTTATTACT  720
   GTCAACAGTA TAGCACCGTG CCGTGGACGT TTGGACAGGG TACCAAGGTG GAGATCAAAC  780
   GAACTGTGGC TGCACCATCT GTCTTCATCT TCCCGCCATC TGATGAGCAG TTGAAATCTG  840
   GAACTGCTTC TGTTGTGTGC CTGCTGAATA ACTTCTATCC CAGAGAGGCC AAAGTACAGT  900
   GGAAGGTGGA TAACGCCCTC CAATCGGGTA ACTCCCAGGA GAGTGTCACA GAGCAGGACA  960
   GCAAGGACAG CACCTACAGC CTCAGCAGCA CCCTGACGCT GAGCAAAGCA GACTACGAGA 1020
   AACACAAAGT CTACGCCTGC GAAGTCACCC ATCAGGGCCT GAGCTCGCCC GTCACAAAGA 1080
   GCTTCAACAG GGGAGAGTGT TAAGCTGATC CTCTACGCCG GACGCATCGT GGCCCTAGTA 1140
   CGCAACTAGT CGTAAAAAGG GTATCTAGAG GTTGAGGTGA TTTTATGAAA AAGAATATCG 1200
   CATTTCTTCT TGCATCTATG TTCGTTTTTT CTATTGCTAC AAACGCGTAC GCTGAGGTTC 1260
   AGCTGGTGGA GTCTGGCGGT GGCCTGGTGC AGCCAGGGGG CTCACTCCGT TTGTCCTGTG 1320
   CAGCTTCTGG CTATACCTTC ACCAACTATG GTATGAACTG GATCCGTCAG GCCCCGGGTA 1380
   AGGGCCTGGA ATGGGTTGGA TGGATTAACA CCTATACCGG TGAACCGACC TATGCTGCGG 1440
   ATTTCAAACG TCGTTTTACT ATTTCTTTAG ACACCTCCGC AAGCACAGTT TACCTGCAGA 1500
   TGAACAGCCT GCGCGCTGAG GACACTGCCG TCTATTACTG TGCAAAGTAC CCCCACTATT 1560
   ATGGGAGCAG CCACTGGTAT TTCGACGTCT GGGGTCAAGG AACCCTGGTC ACCGTCTCCT 1620
   CGGCCTCCAC CAAGGGCCCA TCGGTCTTCC CCCTGGCACC CTCCTCCAAG AGCACCTCTG 1680
   GGGGCACAGC GGCCCTGGGC TGCCTGGTCA AGGACTACTT CCCCGAACCG GTGACGGTGT 1740
   CGTGGAACTC AGGCGCCCTG ACCAGCGGCG TGCACACCTT CCCGGCTGTC CTACAGTCCT 1800
   CAGGACTCTA CTCCCTCAGC AGCGTGGTGA CCGTGCCCTC CAGCAGCTTG GGCACCCAGA 1860
   CCTACATCTG CAACGTGAAT CACAAGCCCA GCAACACCAA GGTCGACAAG AAAGTTGAGC 1920
   CCAAATCTTG TGACAAAACT CACCTCTAGA GTGGCGGTGG CTCTGGTTCC GGTGATTTTG 1980
   ATTATGAAAA GATGGCAAAC GCTAATAAGG GGGCTATGAC CGAAAATGCC GATGAAAACG 2040
   CGCTACAGTC TGACGCTAAA GGCAAACTTG ATTCTGTCGC TACTGATTAC GGTGCTGCTA 2100
   TCGATGGTTT CATTGGTGAC GTTTCCGGCC TTGCTAATGG TAATGGTGCT ACTGGTGATT 2160
   TTGCTGGCTC TAATTCCCAA ATGGCTCAAG TCGGTGACGG TGATAATTCA CCTTTAATGA 2220
   ATAATTTCCG TCAATATTTA CCTTCCCTCC CTCAATCGGT TGAATGTCGC CCTTTTGTCT 2280
   TTAGCGCTGG TAAACCATAT GAATTTTCTA TTGATTGTGA CAAAATAAAC TTATTCCGTG 2340
   GTGTCTTTGC GTTTCTTTTA TATGTTGCCA CCTTTATGTA TGTATTTTCT ACGTTTGCTA 2400
   ACATACTGCG TAATAAGGAG TCTTAATCAT GCCAGTTCTT TTGGCTAGCG CCGCCCTATA 2460
   CCTTGTCTGC CTCCCCGCGT TGCGTCGCGG TGCATGGAGC CGGGCCACCT CGACCTGAAT 2520
   GGAAGCCGGC GGCACCTCGC TAACGGATTC ACCACTCCAA GAATTGGAGC CAATCAATTC 2580
   TTGCGGAGAA CTGTGAATGC GCAAACCAAC CCTTGGCAGA ACATATCCAT CGCGTCCGCC 2640
   ATCTCCAGCA GCCGCACGCG GCGCATCTCG GGCAGCGTTG GGTCCTGGCC ACGGGTGCGC 2700
   ATGATCGTGC TCCTGTCGTT GAGGACCCGG CTAGGCTGGC GGGGTTGCCT TACTGGTTAG 2760
   CAGAATGAAT CACCGATACG CGAGCGAACG TGAAGCGACT GCTGCTGCAA AACGTCTGCG 2820
   ACCTGAGCAA CAACATGAAT GGTCTTCGGT TTCCGTGTTT CGTAAAGTCT GGAAACGCGG 2880
   AAGTCAGCGC CCTGCACCAT TATGTTCCGG ATCTGCATCG CAGGATGCTG CTGGCTACCC 2940
   TGTGGAACAC CTACATCTGT ATTAACGAAG CGCTGGCATT GACCCTGAGT GATTTTTCTC 3000
   TGGTCCCGCC GCATCCATAC CGCCAGTTGT TTACCCTCAC AACGTTCCAG TAACCGGGCA 3060
   TGTTCATCAT CAGTAACCCG TATCGTGAGC ATCCTCTCTC GTTTCATCGG TATCATTACC 3120
   CCCATGAACA GAAATTCCCC CTTACACGGA GGCATCAAGT GACCAAACAG GAAAAAACCG 3180
   CCCTTAACAT GGCCCGCTTT ATCAGAAGCC AGACATTAAC GCTTCTGGAG AAACTCAACG 3240
   AGCTGGACGC GGATGAACAG GCAGACATCT GTGAATCGCT TCACGACCAC GCTGATGAGC 3300
   TTTACCGCAG GATCCGGAAA TTGTAAACGT TAATATTTTG TTAAAATTCG CGTTAAATTT 3360
   TTGTTAAATC AGCTCATTTT TTAACCAATA GGCCGAAATC GGCAAAATCC CTTATAAATC 3420
   AAAAGAATAG ACCGAGATAG GGTTGAGTGT TGTTCCAGTT TGGAACAAGA GTCCACTATT 3480
   AAAGAACGTG GACTCCAACG TCAAAGGGCG AAAAACCGTC TATCAGGGCT ATGGCCCACT 3540
   ACGTGAACCA TCACCCTAAT CAAGTTTTTT GGGGTCGAGG TGCCGTAAAG CACTAAATCG 3600
   GAACCCTAAA GGGAGCCCCC GATTTAGAGC TTGACGGGGA AAGCCGGCGA ACGTGGCGAG 3660
   AAAGGAAGGG AAGAAAGCGA AAGGAGCGGG CGCTAGGGCG CTGGCAAGTG TAGCGGTCAC 3720
   GCTGCGCGTA ACCACCACAC CCGCCGCGCT TAATGCGCCG CTACAGGGCG CGTCCGGATC 3780
   CTGCCTCGCG CGTTTCGGTG ATGACGGTGA AAACCTCTGA CACATGCAGC TCCCGGAGAC 3840
   GGTCACAGCT TGTCTGTAAG CGGATGCCGG GAGCAGACAA GCCCGTCAGG GCGCGTCAGC 3900
   GGGTGTTGGC GGGTGTCGGG GCGCAGCCAT GACCCAGTCA CGTAGCGATA GCGGAGTGTA 3960
   TACTGGCTTA ACTATGCGGC ATCAGAGCAG ATTGTACTGA GAGTGCACCA TATGCGGTGT 4020
   GAAATACCGC ACAGATGCGT AAGGAGAAAA TACCGCATCA GGCGCTCTTC CGCTTCCTCG 4080
   CTCACTGACT CGCTGCGCTC GGTCGTTCGG CTGCGGCGAG CGGTATCAGC TCACTCAAAG 4140
   GCGGTAATAC GGTTATCCAC AGAATCAGGG GATAACGCAG GAAAGAACAT GTGAGCAAAA 4200
   GGCCAGCAAA AGGCCAGGAA CCGTAAAAAG GCCGCGTTGC TGGCGTTTTT CCATAGGCTC 4260
   CGCCCCCCTG ACGAGCATCA CAAAAATCGA CGCTCAAGTC AGAGGTGGCG AAACCCGACA 4320
   GGACTATAAA GATACCAGGC GTTTCCCCCT GGAAGCTCCC TCGTGCGCTC TCCTGTTCCG 4380
   ACCCTGCCGC TTACCGGATA CCTGTCCGCC TTTCTCCCTT CGGGAAGCGT GGCGCTTTCT 4440
   CATAGCTCAC GCTGTAGGTA TCTCAGTTCG GTGTAGGTCG TTCGCTCCAA GCTGGGCTGT 4500
   GTGCACGAAC CCCCCGTTCA GCCCGACCGC TGCGCCTTAT CCGGTAACTA TCGTCTTGAG 4560
   TCCAACCCGG TAAGACACGA CTTATCGCCA CTGGCAGCAG CCACTGGTAA CAGGATTAGC 4620
   AGAGCGAGGT ATGTAGGCGG TGCTACAGAG TTCTTGAAGT GGTGGCCTAA CTACGGCTAC 4680
   ACTAGAAGGA CAGTATTTGG TATCTGCGCT CTGCTGAAGC CAGTTACCTT CGGAAAAAGA 4740
   GTTGGTAGCT CTTGATCCGG CAAACAAACC ACCGCTGGTA GCGGTGGTTT TTTTGTTTGC 4800
   AAGCAGCAGA TTACGCGCAG AAAAAAAGGA TCTCAAGAAG ATCCTTTGAT CTTTTCTACG 4860
   GGGTCTGACG CTCAGTGGAA CGAAAACTCA CGTTAAGGGA TTTTGGTCAT GAGATTATCA 4920
   AAAAGGATCT TCACCTAGAT CCTTTTAAAT TAAAAATGAA GTTTTAAATC AATCTAAAGT 4980
   ATATATGAGT AAACTTGGTC TGACAGTTAC CAATGCTTAA TCAGTGAGGC ACCTATCTCA 5040
   GCGATCTGTC TATTTCGTTC ATCCATAGTT GCCTGACTCC CCGTCGTGTA GATAACTACG 5100
   ATACGGGAGG GCTTACCATC TGGCCCCAGT GCTGCAATGA TACCGCGAGA CCCACGCTCA  5160
   CCGGCTCCAG ATTTATCAGC AATAAACCAG CCAGCCGGAA GGGCCGAGCG CAGAAGTGGT  5220
   CCTGCAACTT TATCCGCCTC CATCCAGTCT ATTAATTGTT GCCGGGAAGC TAGAGTAAGT  5280
   AGTTCGCCAG TTAATAGTTT GCGCAACGTT GTTGCCATTG CTGCAGGCAT CGTGGTGTCA  5340
   CGCTCGTCGT TTGGTATGGC TTCATTCAGC TCCGGTTCCC AACGATCAAG GCGAGTTACA  5400
   TGATCCCCCA TGTTGTGCAA AAAAGCGGTT AGCTCCTTCG GTCCTCCGAT CGTTGTCAGA  5460
   AGTAAGTTGG CCGCAGTGTT ATCACTCATG GTTATGGCAG CACTGCATAA TTCTCTTACT  5520
   GTCATGCCAT CCGTAAGATG CTTTTCTGTG ACTGGTGAGT ACTCAACCAA GTCATTCTGA  5580
   GAATAGTGTA TGCGGCGACC GAGTTGCTCT TGCCCGGCGT CAACACGGGA TAATACCGCG  5640
   CCACATAGCA GAACTTTAAA AGTGCTCATC ATTGGAAAAC GTTCTTCGGG GCGAAAACTC  5700
   TCAAGGATCT TACCGCTGTT GAGATCCAGT TCGATGTAAC CCACTCGTGC ACCCAACTGA  5760
   TCTTCAGCAT CTTTTACTTT CACCAGCGTT TCTGGGTGAG CAAAAACAGG AAGGCAAAAT  5820
   GCCGCAAAAA AGGGAATAAG GGCGACACGG AAATGTTGAA TACTCATACT CTTCCTTTTT  5880
   CAATATTATT GAAGCATTTA TCAGGGTTAT TGTCTCATGA GCGGATACAT ATTTGAATGT  5940
   ATTTAGAAAA ATAAACAAAT AGGGGTTCCG CGCACATTTC CCCGAAAAGT GCCACCTGAC  6000
   GTCTAAGAAA CCATTATTAT CATGACATTA ACCTATAAAA ATAGGCGTAT CACGAGGCCC  6060
   TTTCGTCTTC AA                                                      6072